(19) Europäisches Patentamt
European Patent Office
Office européen des brevets

(11) Veröffentlichungsnummer: **0 206 297**
**B1**

# (12) EUROPÄISCHE PATENTSCHRIFT

(45) Veröffentlichungstag der Patentschrift: **26.07.89**

(51) Int. Cl.⁴: **A 61 K 31/505**, C 07 D 403/04 // (A61K31/505, 31:415)

(21) Anmeldenummer: **86108486.1**

(22) Anmeldetag: **21.06.86**

(54) **4-Amino-2-(imidazolidin-2-on-l-yl)-pyrimidin-5-carbonsäure-(N-(3-trifluormethyl-phenyl)-amide) zur antithrombotischen Prophylaxe und Behandlung sowie ihre Verwendung zur Herstellung von antithrombotisch wirksamen Arzneimitteln.**

(30) Priorität: **27.06.85 DE 3522940**

(43) Veröffentlichungstag der Anmeldung: **30.12.86 Patentblatt 86/52**

(45) Bekanntmachung des Hinweises auf die Patenterteilung: **26.07.89 Patentblatt 89/30**

(84) Benannte Vertragsstaaten: **AT BE CH DE FR GB IT LI LU NL SE**

(56) Entgegenhaltungen: **EP-A- 0 012 361**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Granzer, Ernold, Dr. Dr., Falkensteiner Strasse 24, D-6233 Kelkheim (Taunus) (DE)**
Erfinder: **Kampe, Klaus-Dieter, Dr., Am Rehsteig 1, D-6232 Bad Soden am Taunus (DE)**

**EP 0 206 297 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft die Verwendung von 4-Amino-2-(imidazolidin-2-on-1-yl)-pyrimidin-5-carbonsäure-[N-(3-trifluormethyl-phenyl)-amide] der Formel I

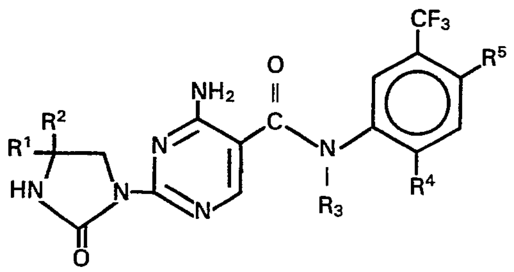

(I)

in der
$R^1$ Wasserstoff, eine $(C_1$-$C_3)$-Alkylgruppe oder eine Vinylgruppe,
$R^2$ eine Methyl- oder Ethylgruppe,
$R^3$ Wasserstoff oder eine Methylgruppe,
$R^4$ und $R^5$, die gleich oder verschieden sind, Wasserstoff, Fluor oder Chlor
bedeuten, wobei $R^4$ und $R^5$ nicht beide gleichzeitig Fluor oder Chlor bedeuten können, sondern, falls $R^4$ Fluor oder Chlor bedeutet, $R^5$ Wasserstoff bedeutet und falls $R^5$ Fluor oder Chlor bedeutet, $R^4$ Wasserstoff bedeutet, sowie deren physiologisch verträgliche Säureadditionssalze zur Herstellung eines Arzneimittels zur Prophylaxe und Therapie von Thrombosen.

Zur Verhinderung der Thrombosen werden gegenwärtig hauptsächlich folgende Medikamente bzw. Medikamentengruppen angewandt:

A. *Heparin und heparinoide* Stoffe, die in die Blutgerinnung eingreifen, die Blutgerinnung hemmen und damit die Ausbildung eines Thrombus verhindern. Ihre Anwendung ist beschränkt, da sie wegen fehlender Resorption bei oraler Gabe nur parenteral anwendbar sind oder in Form von Salben. Ihr bevorzugtes Anwendungsgebiet ist daher die Salbenbehandlung oberflächlicher Venenthrombosen. Die Anwendung zur Verhinderung postoperativer venöser Thrombosen wird jedoch stark eingeschränkt durch ihren Eingriff in die Blutgerinnung, die damit die Gefahr postoperativer Blutungen erheblich vergrößert.

B. *Dikumarole* und andere Verbindungen, die durch Kompetition mit Vitamin K ebenfalls in den Prozeß der Blutgerinnung eingreifen, wurden früher zur Prophylaxe und Nachbehandlung des Myocardinfarktes verwendet. Ihre Anwendung setzt eine regelmäßige Kontrolle der Gerinnungshemmung voraus, wobei zweckmäßigerweise auf eine 30%ige normale Thromboplastinzeit eingestellt wird, um zu verhindern, daß ebenfalls schwere Blutungen auftreten können. Diese Medikamentengruppe kann allerdings oral appliziert werden.

C. Medikamente vom Typ der *Urikonase* bzw. *Streptokinase* wurden in den letzten Jahren vorwiegend zur Behandlung thrombotischer Verursachungen bzw. Komplikationen des Myocardinfarktes verwendet. Auch sie können nur parenteral angewandt werden, wobei ein erfolgreicher Einsatz an eine möglichst schnelle Verabreichung nach erfolgtem Herzinfarkt gebunden ist, um die Gerinnselbildung mittels Plasminogenaktivierung zu verhindern bzw. entstehende Thromben aufzulösen. Neben der systemischen, intravenösen Verabfolgung, die jedoch Probleme der generellen Blutungsneigung aufwirft, kommt ihrer intrakoronaren Applikation per Katheter eine besondere Bedeutung zu.

Aus diesen Hinweisen ergeben sich die Voraussetzungen für eine erfolgreiche Anwendung dieser Medikamente:

1. müssen sie bald nach einem Herzinfarkt gegeben werden,
2. muß ihre Applikation intravenös oder per Katheter intrakoronar erfolgen und
3. ist wegen der systemischen Aktivierung des Plasminogens mit einer verstärkten Blutungsneigung zu rechnen. Im Falle der Streptokinase kommt deren Antigenität als zusätzliche Komplikation in Frage, die eine länger dauernde Therapie oder wiederholte Therapie wegen ihrer Immunogenität beim Menschen nicht zuläßt.

D. *Acetyl-Salicylsäure* und ihre Zubereitungsformen als Plättchenaggregationshemmer haben beim Menschen objektiv nicht zu einem ausreichenden Thromboseschutz geführt; keine der bisherigen Studien konnte nach statistischen Kriterien ihre Wirksamkeit belegen. Dosierungsfragen, die daraus resultieren, daß Acetyl-Salicylsäure als Hemmer der Cyclooxygenase vor Aufspaltung des Weges in die unerwünschte Thromboxanbildung (der Plättchen), als auch in die erwünschte Prostacyclinbildung (in den Gefäßwänden) eingreift, sind möglicherweise die Ursache eines unbewiesenen Thromboseschutzes.

E. Über den Nutzen von gezielten *Thromboxan-Synthetase-Inhibitoren* bzw. *Thromboxan-Antagonisten* sind beim Menschen noch keine ausreichenden Erfahrungen verfügbar. Das Problem der antithrombotischen Therapie mit Plättchenaggregationshemmern ist also ebenfalls nicht frei von Problemen.

Durch den Eingriff der klassischen Antithrombotika vom Typ der Heparinoide bzw. der Dikumarole in die Blutgerinnung besteht bei ihrer Anwendung an Hochdruckpatienten stets die Gefahr der Auslösung schwerer, nicht beherrschbarer zerebraler Blutungen. Daraus resultiert nach wie vor ein Bedarf an Antithrombotika, die Thrombenbildungen verhindern bzw. Thromben auflösen können, ohne systemisch in die Blutgerinnung einzugreifen, zumal der akute Herzinfarkt zu 85% durch Thrombusbildung ausgelöst wird.

In der Europäischen Patentschrift 0 012 361 (entspr. US-PS 4 285 946) werden 4-Amino-2-(imidazolidin-2-on-1-yl)-pyrimidin-5-carbonsäure-[N-(3-trifluormethyl-phenyl)-amide], die aufgrund von anorektischen Wirkungen zur Behandlung von Adipositas und wegen günstiger Wirkungen auf den Lipidstoffwechsel zur Behandlung von Lipidstoffwechselstörungen verwendbar sind, beschrieben.

Es wurde nun überraschenderweise gefunden, daß ein Teil der in der EP-B 0 012 361 (entspr. US-PS 4 285 946) beanspruchten Verbindungen antithrombotisch wirksam ist und daher zur Bekämpfung

und Vorbeugung von allen krankhaften Zuständen, die auf einer Trombenbildung beruhen, geeignet ist.

Die Erfindung betrifft daher die Verwendung von 4-Amino-2-(imidazolidin-2-on-1-yl)-pyrimidin-5-carbonsäure-[N-(3-trifluormethyl-phenyl)-amide] der Formel I sowie deren physiologisch verträgliche Säureadditionssalze zur Herstellung eines Arzneimittels zur Prophylaxe und Therapie von Thrombosen.

An speziellen Verbindungen der allgemeinen Formel I, die antithrombotischen wirksam sind, seien als Beispiele genannt: 4-Amino-2-(4-methyl-4-propyl-imidazolidin-2-on-1-yl)-pyrimidin-5-carbonsäure-[N-(3-trifluormethyl-phenyl)-amid]; 4-Amino-2-(4,4-dimethyl-imidazolidin-2-on-1-yl)-pyrimidin-5-carbonsäure-[N-(2-fluor-5-trifluormethyl-phenyl)-amid]; 4-Amino-2-(4,4-dimethyl-imidazolidin-2-on-1-yl)-pyrimidin-5-carbonsäure-[N-(2-chlor-5-trifluormethyl-phenyl)-amid]; 4-Amino-2-(4-methyl-4-vinyl-imidazolidin-on-1-yl)-pyrimidin-5-carbonsäure-[N-(3-trifluormethyl-phenyl)-amid]; 4-Amino-2-(4,4-dimethyl-imidazolidin-2-on-1-yl)-pyrimidin-5-carbonsäure-[N-methyl-N-(3-trifluormethyl-phenyl)-amid]; 4-Amino-2-(4,4-dimethyl-imidazolidin-2-on-1-yl)-pyrimidin-5-carbonsäure-[N-(4-chlor-3-trifluormethyl-phenyl)-amid]; 4-Amino-2-(4-ethyl-4-methyl-imidazolidin-2-on-1-yl)-pyrimidin-5-carbonsäure-[N-(3-trifluormethyl-phenyl)-amid]; 4-Amino-2-(4-ethyl-imidazolidin-2-on-1-yl)-pyrimidin-5-carbonsäure-[N-(3-trifluormethyl-phenyl)-amid]; 4-Amino-2-(4-methyl-4-propyl-imidazolidin-2-on-1-yl)-pyrimidin-5-carbonsäure-[N-(2-fluor-(oder 2-chlor)-5-trifluormethyl-phenyl)-amid]; sowie deren physiologisch verträgliche Säureadditionssalze.

Besonders geeignet zur Behandlung und Vorbeugung von thrombotischen Zuständen sind 4-Amino-2-(4,4-dimethyl-imidazolidin-2-on-1-yl)-pyrimidin-5-carbonsäure-[N-(3-trifluormethyl-phenyl)-amid] (Formel Ia)

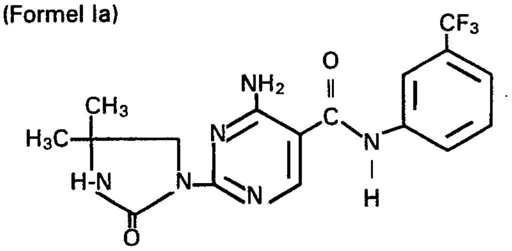

(Ia)

sowie physiologisch unbedenkliche Säureadditionssalze dieser Verbindung.

Als physiologisch unbedenkliche Salze der erfindungsgemäßen Verbindungen der Formel I kommen solche mit anorganischen und organischen Säuren, wie beispielsweise Chlor-, Brom- oder Jodwasserstoff-, Schwefel, Phosphor-, Salpeter-, Benzolsulfon-, Toluolsulfon-, Sulfamin-, Methylschwefel-, Essig-, Propion-, Öl-, Palmitin-, Stearin-, Malon-, Malein-, Bernstein-, Glutar-, Äpfel-, Wein-, Zitronen-, Fumar-, Milch-, Glykol-, Benztrauben-, Benzoe-, Toluyl-, Glutamin-, Furancarbon-, Salicyl- oder Mandelsäure in Betracht. Bevorzugt werden physiologisch verträgliche Salze mit anorganischen Säuren oder sark bis mittelstark sauren Derivaten solcher Säuren.

Die Herstellung der gemäß vorliegender Erfindung zu verwendenden Verbindungen der allgemeinen Formel I ist in der Europäischen Patentschrift 0 012 361 (entspr. US-PS 4 285 946) beschrieben. Sie erfolgt zweckmäßigerweise nach der unter «b») beschriebenen Synthesemethode.

Beispielsweise kann 4-Amino-2-(4,4-dimethyl-imidazolidin-2-on-1-yl)-pyrimidin-5-carbonsäure-[N-(3-trifluormethyl-phenyl)-amid] (Formel Ia) durch Umsetzung von 1-Amidino-4,4-dimethyl-imidazolidin-2-on bzw. dessen Säureadditionssalzen mit 2-Cyano-3-ethoxy-acrylsäure-[N-(3-trifluormethyl-phenyl)-amid], gemäss nachstehendem Formelbild, hergestellt werden.

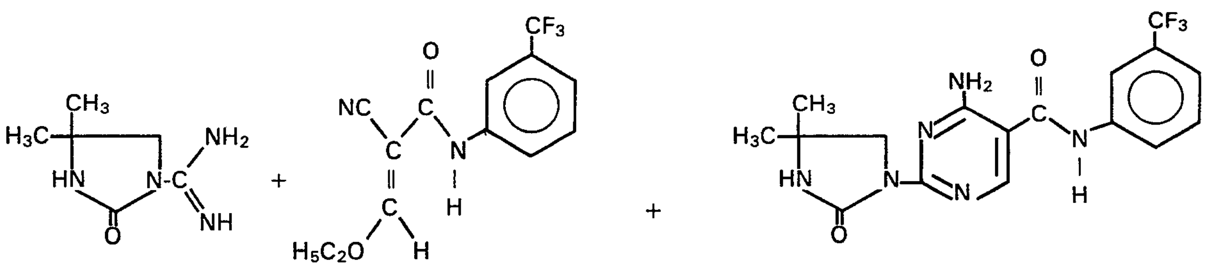

(Ia)

Die Synthese der Ausgangsverbindungen ist in der Europäischen Patentschrift 0 012 361 beschrieben worden.

Die antithrombotische Wirkung der Verbindungen der Formel I konnte in folgenden Untersuchungen festgestellt werden.

In der modifizierten Anordnung eines erstmals von Meng angewandten und in Arzneimittel-Forschung 29, 54 (1979) ausführlich beschriebenen Tiermodells, werden durch eine lokale Gefäßschädigung an A. carotis und V. jugularis von Ratten durch Unterkühlung des Gefäßes und Druckausübung an diesen Gefäßstellen Thromben erzeugt. Durch Vorbehandlung der Tiere mit den Verbindungen der Formel I werden gegenüber einer Kontrollgruppe entweder mehr Tiere von Thromben freigehalten oder das Ausmaß (durch Gewicht definiert) der entstehenden Thromben deutlich reduziert; gegenüber einer mitgeführten Kontrollgruppe ist also das Durchschnittsgewicht der Thromben in der mit dem Präparat behandelten Gruppe deutlich niedriger als in der Kontrollgruppe.

Männliche Ratten erhielten an 7 aufeinanderfolgenden Tagen per Schlundsonde die in der Tabelle angegebene Menge der zu prüfenden Verbindung, die Kontrollgruppe nur die gleiche Menge Lösungsmittel (Polyethylenglykol 400). Die letzte Applikation erfolgte 20 Stunden vor Versuchsende, d.h. vor der Operation zur Erzeugung von Thromben mit der Kryozange. In den in der Tabelle mit * bezeichneten Versuchen wurde nur an 6 aufeinanderfolgenden Tagen mit letzter Applikation 1 Stunde vor Versuchsende behandelt. In der Versuchsreihe A) erhielten die Tiere normale Diät enthaltend Protein, Fett, Fasern, Mineralstoffe, Spurenelemente und Vitamine (z.B. Altromin, Kontroll-Diät, Hersteller: Altromin GmbH), in der Versuchsreihe B) erhielten sie während der Versuchszeit eine atherogene Spezialität ad libitum, die 5% Schweineschmalz, 1% Cholesterin, 0,3% Taurocholsäure, und 0,1% Propylthiouracil im Normalfutter Altromin enthielt. Die Thromben wurden 4 Stunden vor der Thrombenentnahme aus den Gefäßen durch Unterkühlung der Gefäßwand erzeugt. Dazu wurden die Ratten mit Äther ca. 2 bis 3 Minuten narkotisiert. Nach einem paramedianen Hautschnitt und Spreizung des subkutanen Gewebes wurden A. carotis und V. jugularis auf eine Länge von ca. 2 cm freipräpariert.

Mit einer Kryozange, die durch Durchfluß mit kaltem Methanol auf eine Temperatur von −15° gebracht wurde, drückte man 2 Minuten lang mit einer Kraft von 200 g auf die freigelegte A. carotis über eine Länge von 12 mm. Distal von dem geschädigten Arterienstück wurde eine Silberklammer angebracht, die in dem Tier verblieb. Danach wurde die Wunde mit 2 Wundklammern verschlossen. 4 Stunden nach dem Eingriff wurden die Tiere nacheinander mit 50 mg/kg Nembutal i.p. narkotisiert, wobei zur Verhinderung einer akuten Blutgerinnung gleichzeitig 2500 E/kg Heparin i.p. injiziert wurden. Nach 10 Minuten wurde die Wunde geöffnet und ein ca. 15 mm grosses Arterienstück mit dem entstandenen Thrombus entnommen. Das entnommene Arterienstück wurde mit einem Längsschnitt geöffnet und der anhaftende Thrombus abpräpariert, in Kochsalzlösung und anschliessend Wasser gespült und gewaschen, wenige Sekunden auf gut saugendem Filterpapier abgetupft und mit einer elektronischen Mikrowaage gewogen. In prinzipiell ähnlicher Weise wurde die V. jugularis zwecks Erzeugung venöser Thromben behandelt und die Hemmung der venösen Thrombenbildung bestimmt. Das mittlere Gewicht der Thromben aller Tiere einer mit Prüfsubstanz behandelten Tiergruppe wurde mit dem mittleren Thrombengewicht aller Tiere der Kontrollgruppe verglichen (wobei fehlende Thromben mit dem Gewicht 0 in die Berechnung einging). Wie aus der Tabelle ersichtlich, wurde in dem angewandten Dosierungsschema die Thrombenbildung, vorzugsweise die arterielle, durch 4-Amino-2-(4,4-dimethyl-imidazolidin-2-on-1-yl)-pyrimidin-5-carbonsäure-[N-(3-trifluor-methyl-phenyl)-amid] (Formel Ia) verhindert, wobei die arterielle Thrombenbildung bis zu einer Dosis von 0,3 mg/kg/Tag gehemmt wurde, die venöse Thrombenbildung war nur in höherer Dosierung deutlich zu hemmen.

Tabelle

| | Dosis mg/kg/ Tag | Veränderung des Thrombus-Feuchtgewichts in der A. carotis | in der V. jugularis gegenüber der Kontrollgruppe |
|---|---|---|---|
| A) 4-Amino-2-(4,4-dimethyl-imidazolidin-2-on-1-yl)-pyrimidin-5-carbonsäure-[N-(3-trifluormethyl-phenyl)-amid] | 30 | −89% | |
| | 0,3* | −37% | −18% |
| Acetylsalicylsäure | 10 | −21% | |
| B) 4-Amino-2-(4,4-dimethyl-imidazolidin-2-on-1-yl)-pyrimidin-5-carbonsäure-[N-(3-trifluormethyl)-phenyl)-amid] | 30 | −79% | −68% |
| | 10 | −63% | − 6% |
| | 3 | −71% | − 1% |
| | 1 | −47% | |
| | 0,3* | −18% | |

Die Anwendung der Verbindung der Formel Ia verursacht keinen Eingriff in die Gerinnung, was durch eine 5wöchige Gabe von 10 und 30 mg/kg/Tag an Hunde und Prüfung des Gerinnungsverhaltens durch Prothrombinzeit, Thrombinzeit und Thrombelastogramm nachgewiesen wurde; in keinem Fall ergaben sich Veränderungen, die für eine beeinträchtigte Gerinnung (d.h. Gerinnungsverzögerung), sprachen.

Bei der Herstellung von Arzneimitteln gemäss vorliegender Erfindung können neben Verbindungen der Formel I, die in Form der freien Basen und/oder in Form ihrer pharmazeutisch annehmbaren Säureadditionssalze als Arzneimittel Verwendung finden, auch pharmazeutisch unbedenkliche Zusatzstoffe, wie Verdünnungsmittel und/oder Trägermaterialien verwendet werden. Hierunter sind physiologisch unbedenkliche Substanzen zu verstehen, die nach dem Vermischen mit dem Wirkstoff diesen in eine für die Verabreichung geeignete Form bringen.

Geeignete feste oder flüssige galenische Zubereitungsformen sind beispielsweise Tabletten, Dragees, Pulver, Kapseln, Suppositorien, Sirupe, Emulsionen, Suspensionen, Tropfen oder injizierbare Lösungen sowie Präparate mit protrahierter Wirkstoff-Freigabe. Als häufig verwendete Trägerstoffe bzw. Verdünnungsmittel seien z.B. verschiedene Zucker oder Stärkearten, Cellulosederivate, Magnesiumcarbonat, Gelatine, tierische und pflanzliche Öle, Polyethylenglykole, Wasser oder andere geeignete Lösungsmittel sowie wasserhaltige Puffermittel, die durch Zusatz von Glucose oder Salzen isotonisch gemacht werden können, genannt. Außerdem können gegebenenfalls oberflächenaktive Mittel, Farb- und Geschmacksstoffe, Stabilisatoren, sowie Konservierungsmittel als weitere Zusatzstoffe in den erfindungsgemäßen Arzneimittelzubereitungen Verwendung finden.

Die Präparate können oral, rectal oder parenteral verabreicht werden.

Vorzugsweise können die Präparate in Dosierungseinheiten hergestellt werden. Insbesondere Tabletten, Kapseln, Suppositorien und Ampullen stellen Beispiele für geeignete Dosierungseinheiten dar. Jede Dosierungseinheit, insbesondere für die orale Applikation, kann bis zu 1000 mg, bevorzugt jedoch 15 bis 200 mg, des aktiven Bestandteils enthalten. Jedoch können auch darüber oder darunter liegende Dosierungseinheiten verwendet werden, die gegebenenfalls vor Verabreichung zu teilen bzw. zu vervielfachen sind. Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten von teilchenförmigem Material in geeignete Polymere, Wachse oder dergleichen.

Die parenterale Verabreichung kann unter Verwendung flüssiger Dosierungsformen, wie steriler Lösungen und Suspensionen erfolgen, die für die intramuskuläre, oder subkutane Injektion bestimmt sind. Derartige Dosierungsformen werden durch Lösen bzw. Suspendieren einer abgemessenen Wirkstoffmenge in einem geeigneten physiologisch unbedenklichen Verdünnungsmittel wie beispielsweise einem wäßrigen oder öligen Medium und Sterilisierung der Lösung bzw. der Suspension, gegebenenfalls unter Mitverwendung von geeigneten Stabilisatoren, Emulgatoren und/oder Konservierungsmitteln, hergestellt.

Die orale Applikationsform ist bevorzugt und stellt eine wesentliche Erleichterung in der Thrombosebehandlung dar.

Die pharmazeutischen Präparate werden nach allgemein üblichen Verfahren hergestellt.

*Beispiel 1: Tabletten*

Tabletten, die für die orale Verabreichung geeignet sind und die nachfolgend genannten Bestandteile enthalten, werden auf an sich bekannte Weise hergestellt, indem man Wirk- und Hilfsstoffe granuliert und anschließend zu Tabletten verpreßt. Diese Tabletten eignen sich zur antithrombotischen Behandlung in einer Dosis von einer Tablette 2 - 4 mal täglich.

| *Bestandteile* (pro Tablette) | *Gewicht (mg)* |
|---|---|
| 4-Amino-2-(4,4-dimethyl-imidazolidin-2-on-1-yl)-pyrimidin-5-carbonsäure-[N-(3-trifluormethyl-phenyl)-amid] | 50 mg |
| Milchzucker | 100 mg |
| Maisstärke | 30 mg |
| Talkum | 3 mg |
| kolloidales Siliziumdioxid | 3 mg |
| Magnesiumstearat | 2 mg |

*Beispiel 2: Kapseln*

Kapseln, die für die orale Verabreichung geeignet sind, enthalten die nachfolgend genannten Bestandteile und können auf an sich bekannte Weise hergestellt werden, indem man Wirk- und Hilfsstoffe vermischt und in Gelatinekapseln abfüllt. Diese Kapseln dienen zur antithrombotischen Behandlung in einer Dosis von einer Kapsel 2 - 4 mal täglich.

| *Bestandteile* (pro Kapsel) | *Gewicht (mg)* |
|---|---|
| 4-Amino -2-(4,4-dimethyl-imidazolidin-2-on-1-yl)-pyrimidin-5-carbonsäure-[N-(3-trifluormethyl-phenyl)-amid] | 50 mg |
| Milchzucker | 100 mg |
| Maisstärke | 30 mg |
| Talkum | 3 mg |
| kolloidales Siliziumdioxid | 3 mg |
| Magnesiumstearat | 2 mg |

**Patentansprüche**

1. Verwendung von 4-Amino-2-(imidazolidin-2-on-1-yl)-pyrimidin-5-carbonsäure-[N-(3-trifluormethyl-phenyl)-amiden] der Formel I

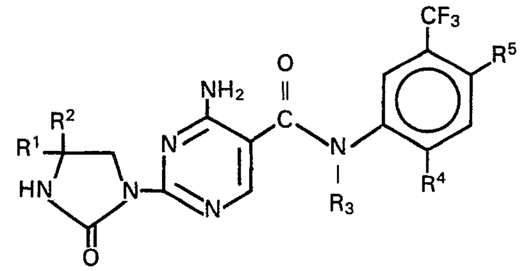

(I)

in der

$R^1$ Wasserstoff, eine ($C_1$-$C_3$)-Alkylgruppe oder eine Vinylgruppe,

$R^2$ eine Methyl- oder Ethylgruppe,

$R^3$ Wasserstoff oder eine Methylgruppe,

$R^4$ und $R^5$, die gleich oder verschieden sind, Wasserstoff, Fluor oder Chlor

bedeuten, wobei $R^4$ und $R^5$ nicht beide gleichzeitig Fluor oder Chlor bedeuten können, sondern, falls $R^4$ Fluor oder Chlor bedeutet, $R^5$ Wasserstoff bedeutet und falls $R^5$ Fluor oder Chlor bedeutet, $R^4$ Wasserstoff bedeutet, sowie von deren physiologisch verträgliche Säureadditionssalzen zur Herstellung eines Arzneimittels zur Prophylaxe und Therapie von Thrombosen.

2. Verwendung von 4-Amino-2-(4,4-dimethylimidazolidin-2-on-1-yl)-pyrimidin-5-carbonsäure-[N-(3-trifluormethyl-phenyl)-amid] sowie von dessen physiologisch verträglichen Säureadditionssalzen gemäß Anspruch 1 zur Herstellung eines Arzneimittels zur Prophylaxe und Therapie von Thrombosen.

3. Verwendung von Verbindungen der Formel I gemäss Anspruch 1 sowie von deren physiologisch verträglichen Säureadditionssalzen zur Herstellung eines Arzneimittels zur oralen Anwendung bei der Prophylaxe und Therapie von Thrombosen.

**Claims**

1. The use of 4-amino-2-(imidazolidin-2-on-1-yl)-5-(3-trifluormethylphenylaminocarbonyl)pyrimidines of the formula I

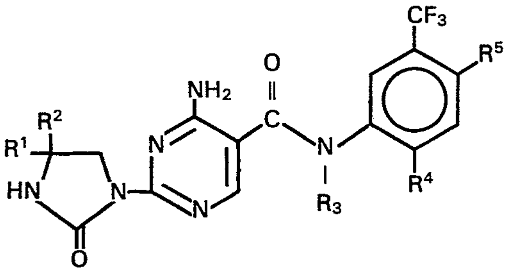

(I)

in which

$R^1$ denotes hydrogen, a $(C_1\text{-}C_3)$-alkyl group or vinyl group

$R^2$ denotes a methyl or ethyl group,

$R^3$ denotes hydrogen or a methyl group,

$R^4$ and $R^5$, which are identical or different, denote hydrogen, fluorine or chlorine,

it not being permissible for $R^4$ and $R^5$ both simultaneously to denote fluorine or chlorine, but $R^5$ denoting hydrogen if $R^4$ denotes fluorine or chlorine, and $R^4$ denoting hydrogen if $R^5$ denotes fluorine or chlorine, and of their physiologically tolerated acid addition salts for the preparation of a medicament for the prophylaxis and therapy of thromboses.

2. The use of 4-amino-2-(4,4-dimethylimidazolidin-2-on-1-yl)-5-(3-trifluoromethylphenylaminocarbonyl)pyrimidine and of its physiologically tolerated acid addition salts as claimed in claim 1 for the preparation of a medicament for the prophylaxis and therapy of thromboses.

3. The use of compounds of the formula I as claimed in claim 1 and of their physiologically tolerated acid addition salts for the preparation of a medicament for oral use for the prophylaxis and therapy of thromboses.

## Revendications

1. Utilisation de 4-amino-2-(imidazolidine-2-one-1-yl)-pyrimidine-5-[N-(3-trifluorométhyl-phényl)-carboxamides] de formule I

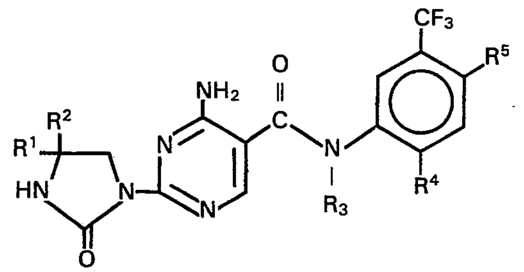

(I)

dans laquelle

$R^1$ représente un atome d'hydrogène ou un groupe alkyle en $C_1\text{-}C_3$ ou le groupe vinyle

$R^2$ représente le groupe méthyle ou éthyle,

$R^3$ représente un atome d'hydrogène ou le groupe méthyle,

$R^4$ et $R^5$, qui sont identiques ou différents, représentent un atome d'hydrogène, de fluor ou de chlore,

$R^4$ et $R^5$ ne pouvant pas représenter tous les deux en même temps des atomes de fluor ou de chlore, mais, dans le cas où $R^4$ représente un atome de fluor ou de chlore, $R^5$ représente un atome d'hydrogène, et dans le cas où $R^5$ représente un atome de fluor ou de chlore, $R^4$ représente un atome d'hydrogène, ainsi que leurs sels d'addition physiologiquement acceptables avec des acides, pour la fabrication d'un médicament pour le traitement et la prophylaxie de thromboses.

2. Utilisation du 4-amino-2-(4,4-diméthylimidazolidine-2-one-1-yl)-pyrimidine-5-[N-(3-trifluorométhyl-phényl)-carboxamide] ainsi que des sels d'addition physiologiquement acceptables de celui-ci avec des acides selon la revendication 1, pour la fabrication d'un médicament pour la prophylaxie et le traitement de thromboses.

3. Utilisation des composés de formule I selon la revendication 1, ainsi que de leurs sels d'addition physiologiquement acceptables avec des acides, pour la fabrication d'un médicament pour l'administration orale, dans la prophylaxie et le traitement de thromboses.